# EUROPÄISCHE PATENTSCHRIFT

(11) **EP 1 681 345 B1**
(45) Veröffentlichungstag und Bekanntmachung des Hinweises auf die Patenterteilung: **01.01.2014**
(21) Anmeldenummer: 06001034.5
(22) Anmeldetag: 18.01.2006
(51) Int. Cl.: C12N 1/02, C12N 11/02, C12N 11/14, A23L 3/00, A61L 2/00

(54) **Verfahren und Kit zur Sterilisierung von Mikroorganismen enthaltenden Flüssigkeiten**
Method and kit for the sterilization of fluids containing microorganisms
Procédé et kit pour la stérilisation de liquides comprenant des microorganismes

(30) Priorität: 18.01.2005 DE 102005002342
(43) Veröffentlichungstag der Anmeldung: 19.07.2006
(73) Patentinhaber: GEN-Institut für Angewandte Laboranalysen GmbH, 53840 Troisdorf (DE); Bitburger Brauerei Th.Simon GmbH, 54634 Bitburg (DE)
(72) Erfinder: Schönling, Jutta, 50823 Köln (DE); Mücher, Gabriele, 53859 Niederkassel (DE)
(74) Vertreter: Pfenning, Meinig & Partner GbR

(56) Entgegenhaltungen:
- WO-A-98/51693
- WO-A-03/033698
- WO-A1-02/34075
- FR-A- 2 440 402
- KLEIN J ET AL: "Immobilization of microbial cells by adsorption" JOURNAL OF BIOTECHNOLOGY, ELSEVIER SCIENCE PUBLISHERS, AMSTERDAM, NL, Bd. 16, Nr. 1-2, Oktober 1990 (1990-10), Seiten 1-15, XP002365336 ISSN: 0168-1656

## Beschreibung

Die Erfindung betrifft die Verwendung eines Verfahrens unspezifischen Abtrennung und Analyse von Mikroorganismen aus großvolumigen Flüssigkeiten. Hierdurch wird es erstmalig möglich, auch große Flüssigkeitsvolumina im Liter-Maßstab zu sterilisieren. Weiterhin ermöglicht das Verfahren die Analytik der isolierten Mikroorganismen. Die erfindungsgemäße Verwendung des Verfahrens ist insbesondere zur unspezifischen Abtrennung von Bakterien, Hefen, Viren, Pilzen und Sporen aus großvolumigen Flüssigkeiten geeignet.

Für die Sterilisation von Flüssigkeiten wird in der Regel eine Behandlung unter Druck und hoher Temperatur, sog. Autoklavieren, oder eine Sterilfiltration durchgeführt. Erstere Behandlung führt zwar zum Absterben der Mikroorganismen aber nicht zur Entfernung der DNA, die sich in nachfolgenden Applikationen als irreführend bemerkbar machen kann, z.B. bei der PCR, während die Sterilfiltration nur für wässrige Lösungen mit begrenztem Volumen anwendbar ist.

Für die direkte Isolierung von Mikroorganismen stehen derzeit nur für kleinste Flüssigkeitsmengen im Milliliter-Maßstab Verfahren zur Verfügung (DE 101 49 803 A1), während für große Mengen im Liter-Maßstab keine oder nur Verfahren mit großem Aufwand an Zeit, Geld und Apparaten zur Verfügung stehen. Als Standardmethode bei filtrierbaren Lösungen großer Volumina dient eine zeitaufwendige Membranfiltration (WO/0152968). Die in der Routine eingesetzten Membranfilter für Bakterien (0,45 µm) sind für kleine Bakterien und Viren noch durchlässig und damit auch nicht sicher. Für nicht filtrierbare Flüssigkeiten steht augenblicklich noch keine zuverlässige Isolierungsmethode zur Verfügung. Die Isolierung von Viren aus größeren Volumina stellt heute noch ein ganz großes Problem dar. Die Filter müssen eine so kleine Probengröße haben, dass wässrige Lösungen die Filter sehr schnell zusetzen.

Bei kleinen Volumina wird zur Zeit die Zentrifugation eingesetzt. Die Probennahme reduziert sich hier auf ein kleines Probenvolumen, in dem geringe mikrobielle und virale Bestandteile eventuell nicht erfasst werden. Die Nachweisgrenze liegt demzufolge weit über dem gewünschten Wert von 1 Keim pro Liter. In beiden Fällen erfolgt eine mehrtägige Anreicherung verbunden mit zeitraubenden und aufwendigen physiologischen und kulturellen Anreicherungstests. Eine Lösungsmöglichkeit zur Umgehung der Zentrifugation belegt das Patent DE 101 49 803 A1, in dem Bakterien aus geringen Volumen Kulturmedien an flüssige magnetisierte Polymere gebunden werden.

In der Patentanmeldung WO 98/51693 A wird eine im Labormaßstab durchgeführte Isolierung von Zellen (z.B. Bakterienzellen) aus kleinen Flüssigkeitsvolumina (Milliliterbereich) beschrieben, wobei die lolierung durch unspezifische Bindung der Zellen an ein festes Trägermaterial erfolgt und die Zellen nach der Isolierung lysiert werden um ihre DNA zu untersuchen.

Die FR-A-2 440 402 offenbart ein Verfahren im Labormaßstab zur Sterilisierung von Flüssigkeiten, wobei Mikroorganismen aus den Flüssigkeiten isoliert werden indem sie an feste Tägermaterialien gebunden werden.

In der WO 02/34075 A1 ist ein Verfahren zur Sterilisierung großvolumiger Flüssigkeiten offenbart, wobei in diesem Verfahren die Flüssigkeit zunächst auf über 60°C erhitzt wird und anschließend ein elektrisches Feld angelegt wird.

Die Nachteile heutiger Verfahren sind zusammenfassend:
- keine Direktisolierung aus großen Probenvolumina möglich,
- mehrtägige Anreicherungen in diversen Selektivmedien,
- zeitaufwendige und unsichere Membranfiltrationen,
- kleines Probenvolumen verbunden mit dem Risiko geringe Konzentrationen nicht aufzuspüren,
- magnetisierte Polymere kostenintensiv, insbesondere für große Volumina ungeeignet
- keine praktikable Lösung für die Isolierung von Viren ohne Anreicherung
- kein Verfahren zur Sterilisierung durch Abreicherung von Mikroorganismen in großem Maßstab und anschließender DNA-Freiheit von Lösung.

Ausgehend hiervon war es Aufgabe der vorliegenden Erfindung, ein Verfahren bereitzustellen, das eine unspezifische Abtrennung und Analyse von Mikroorganismen aus großvolumigen Flüssigkeitsmengen ermöglicht, wobei dennoch eine einfache und schnelle Handhabung gewährleistet ist, um eine kostengünstige Verfahrensführung zu ermöglichen.

Diese Aufgabe wird durch die Verwendung des Verfahrens mit den Merkmalen das Anspruchs 1 gelöst. Die weiteren abhängigen Ansprüche zeigen vorteilhafte Weiterbildungen auf.

Erfindungsgemäß wird eine Verwendung eines Verfahrens zur unspezifischen Abtrennung und Analyse von Mikroorganismen aus großvolumigen Flüssigkeiten bereitgestellt. Dieses beruht im Wesentlichen auf den folgenden Schritten:
a) In der Flüssigkeit wird ein Trägermaterial aus Polymeren, Glas oder Keramik, dessen Oberfläche zur unspezifischen Adsorption von in der Flüssigkeit enthaltenden Mikroorganismen durch Anbindung funktioneller chemischer Gruppen chemisch funktionalisiert wurde, homogen verteilt. Der pH-Wert der Flüssigkeit wird dabei in Abhängigkeit von der Art der Mikroorganismen so eingestellt, dass eine maximale Adsorption der Mikroorganismen an dem Trägermaterial gewährleistet ist.
b) Die mit dem Trägermaterial versehene Flüssigkeit wird bei einer Temperatur im Bereich von 10 bis 37 °C geschüttelt, bis die Chemisorption der Mikroorganismen aufgrund kovalenter und/oder ionischer Wechselwirkungen am Trägermaterial abgeschlossen ist.
c) Anschließend erfolgt die Abtrennung des mit den Mikroorganismen beladenen Trägermaterials von der Flüssigkeit.
d) Gegebenenfalls erfolgt eine Anreicherung des mit Mikroorganismen beladenen Trägermaterials in geeignetem Nährmedium.
e) Regenerierung des mit den Mikroorganismen beladenen Trägermaterials durch Lyse und gleichzeitige Isolierung einer DNA enthaltenden Lösung.
f) Analyse der DNA enthaltenden Lösung.

Mit der Verwendung des hier beschriebenen Verfahrens gelingt es erstmalig, Mikroorganismen und Viren unspezifisch an oberflächenfunktionalisierte Polymerpartikel in Festkörperform aus großen Flüssigkeitsvolumen zu binden. Diese Flüssigkeiten werden dadurch einerseits sterilisiert (DNA-Freiheit), und andererseits werden die isolierten Mikroorganismen für einen Schnellnachweis zugänglich gemacht. Die Verwendung der Methode ermöglicht es, Mikroorganismen schon im Anfangsstadium ihrer Vermehrung in extrem niedrigen Konzentrationen sehr schnell und sicher zu erfassen. Die Verwendung des Verfahrens ist kostengünstig, einfach und vielseitig anwendbar.

Die Zugabe des Trägermaterials erfolgt zu einer definierten Menge der Flüssigkeit. Für eine effiziente Bindung der Mikroorganismen an das Trägermaterial muss ein definierter pH-Wert vorliegen. Der pH-Wert wird bei Bedarf vorher mit bestimmten Pufferlösungen eingestellt. Für das erfindungsgemäße Verfahren ist ein definierter pH-Wert der Flüssigkeit entscheidend. Für eine effiziente Bindung der Mikroorganismen müssen bestimmte Anforderungen an die Lösungen gestellt werden und diese bei Nichterfüllung gegebenenfalls modifiziert werden. Die Konzentration des Trägermaterials ist dabei abhängig von der Anzahl und Art der Mikroorganismen sowie dem Volumen der Flüssigkeit. Sie liegen in einem Konzentrationsbereich vor, in dem eine ausreichende Bindung der Mikroorganismen an das Trägermaterial gewährleistet ist.

Das Schütteln der Flüssigkeit und des Trägermaterials erfolgen bei einer definierten Temperatur im Bereich von 10 bis 37 °C und definierten Bewegungen. Durch die Wahl geeigneter Inkubationstemperaturen und Inkubationszeiten kann während der Bindungsphase auch eine kurzfristige Voranreicherung der Mikroorganismen durchgeführt werden. Dadurch wird die Sensitivität für einzelne Mikroorganismen erhöht.

Für die Abtrennung des Trägermaterial-Mikroorganismengemisches von der Flüssigkeit stehen die aus dem Stand der Technik bekannten Trennverfahren zur Verfügung. Die einzige Einschränkung hierzu besteht darin, dass zum einen die Mikroorganismen während des Abtrennens am Trägermaterial gebunden bleiben, mögliche Inhibitoren aus der Flüssigkeit entfernt werden und kein Trägermaterial verloren geht.

Als Trägermaterial finden Polymere, Glas oder Keramik Verwendung.

Vorzugsweise wird das Trägermaterial in Form eines Pulvers, eines Granulats, einer Folie, einer Membran, eines Sinterkörpers oder eines Schaums eingesetzt. Das Trägermaterial kann dabei vorzugsweise hydrophil oder hydrophob funktionalisiert sein. Die Funktionalisierung kann vorzugsweise so durchgeführt werden, dass die funktionellen Gruppen direkt oder über einen Spacer an das Trägermaterial gebunden sind.

Beruht die Chemisorption auf einer chemischen Wechselwirkung, so sind die funktionellen Gruppen bevorzugt ausgewählt aus der Gruppe bestehend aus Amin-, Carboxyl, Carboxylat-, Sulphonsäure-, Sulphonat-, Phosphatgruppen und Proteinen.

Beruht die Chemisorption hingegen auf einer kovalenten Wechselwirkung, so sind die funktionellen Gruppen bevorzugt ausgewählt aus der Gruppe bestehend aus Aktivester, Säureamiden, Epoxiden, Halogeniden, Säurehalogeniden und C-C-Mehrfachbindungen.

Das funktionalisierte Trägermaterial kann dabei neutralen, kationischen oder auch anionischen Charakter aufweisen. Dies kann in Abhängigkeit von den zu isolierenden Mikroorganismen beliebig eingestellt werden.

Das Inkontaktbringen des Trägermaterials mit der Flüssigkeit erfolgt vorzugsweise durch eine Dispersion des Trägermaterials in dem flüssigen Medium. Nach einer weiteren bevorzugten Variante kann das Trägermaterial aber auch in Form eines porösen Körpers vorliegen, durch dessen Poren dann das flüssige Medium strömt.

Die Abtrennung des Trägermaterials vom flüssigen Medium erfolgt vorzugsweise mittels Filtration, Sedimentation oder Absieben.

Das abgetrennte und mit Mikroorganismen beladene Trägermaterial kann vorzugsweise in einem zusätzlichen Schritt einer Voranreicherung in geeigneten Nährmedien unterworfen werden.

Die erfindungsgemäße Verwendung des Verfahrens sieht vor, dass das Gemisch aus Trägermaterial und Mikroorganismen einer direkten Lyse bei definierten pH-Werten mit verschiedenen Lysepuffern unterworfen wird. Die Auswahl des Lysepuffers ist dabei abhängig von den zu isolierenden Mikroorganismen. Hierzu erfolgt eine Zugabe von Lysepuffern und gegebenenfalls Enzymen zu dem von der Flüssigkeit abgetrennten Gemisch aus Trägermaterial und Mikroorganismen. Im Anschluss erfolgt eine Inkubation des Gemisches mit Lysepuffern und gegebenenfalls Enzymen. Die geeignete Inkubationstemperatur und die Enzymauswahl für die Lyse sind abhängig von der Art des zu isolierenden Mikroorganismus oder den zu isolierenden Mikroorganismen. Die Auswahl der Parameter erlaubt eine erhöhte Ausbeute an DNA. Im Anschluss wird dann die DNA-enthaltende Lösung vom Trägermaterial abgetrennt, wobei auch hier aus dem Stand der Technik bekannte Trennungsverfahren angewendet werden können.

Dieses System erlaubt es dem Anwender, Mikroorganismen und Viren aus den zu untersuchenden Flüssigkeiten direkt herauszuisolieren und anzureichern. Einerseits kann die Flüssigkeit dadurch sterilisiert werden und andererseits kann die DNA der isolierten Mikroorganismen durch mitgelieferte Lysepuffer und Enzyme extrahiert und die Spezies mit nachfolgenden, bereits etablierten molekularbiologischen Methoden identifiziert werden.
Mit der erfindungsgemäßen Verwendung des Verfahrens können beliebige Mikroorganismen aus großvolumigen Flüssigkeiten, insbesondere Bakterien, Hefen, Viren, Pilzen und Sporen, unspezifisch abgetrennt werden. Die Anwendungsfelder sind hier grundsätzlich nicht eingeschränkt, sondern betreffen sämtliche Gebiete, in denen Mikroorganismen eine Rolle spielen. Beispielsweise zählen hierfür mikrobiologische Qualitätskontrollen und Qualitätsprüfungen, insbesondere bei der Herstellung von Getränken und Flüssigkeiten, bei der Herstellung von Kulturmedien für medizinische und lebensmitteltechnische Anwendungen, bei der Herstellung von Zellkulturen, bei der Herstellung von Medikamenten, bei der Wasseraufbereitung in Klärwerken und Schwimmbädern und im Trinkwasserbereich.

Anhand des nachfolgenden Beispiels soll die Verwendung des erfindungsgemäßen Verfahrens näher erläutert werden, ohne diese auf die hier genannte spezielle Ausführungsform einschränken zu wollen.

### Beispiel

Ein Liter Bier wird mit einer definierten Menge an Lactoacillus brevis-Keimen beimpft und mit 500 mg oberflächenfunktionalisiertem Trägermaterial versetzt. Das Trägermaterial ist dabei mit Polyethylenimin-Gruppen funktionalisiert. Das Bier wird im Anschluss mit einem Überkopfschüttler 2 h bei 29 °C geschüttelt. Im Anschluss wird das beladene Trägermaterial mit einer speziell angefertigten Siebapparatur unter mehrmaligem Spülen mit sterilem Wasser (pH 5,0) abgesiebt.

Das so mit den Mikroorganismen beladene Trägermaterial kann aus dem Sieb herausgelöst werden und zur kulturellen Kurzanreicherung in ein Kulturmedium, z.B. ein MRS-Medium, gegeben, und für ca. 16 bis 24 h bei 29 °C unter Schütteln inkubiert werden. Zur Direktisolierung der Bakterien-DNA werden anschließend die auf dem Trägermaterial gebundenen Bakterien lysiert und die DNA mit gängigen Methoden isoliert.

## Patentansprüche

1. Verwendung eines Verfahrens zur unspezifischen Abtrennung und Analyse von Mikroorganismen aus großvolumigen Flüssigkeiten, bei dem
a) in der Flüssigkeit ein Trägermaterial aus Polymeren, Glas oder Keramik, dessen Oberfläche zur unspezifischen Adsorption von in der Flüssigkeit enthaltenden Mikroorganismen durch Anbindung funktioneller chemischer Gruppen chemisch funktionalisiert wurde, homogen verteilt wird, wobei der pH-Wert der Flüssigkeit in Abhängigkeit von der Art der Mikroorganismen eingestellt wird,
b) Schütteln der Flüssigkeit und des Trägermaterials bei einer Temperatur im Bereich von 10 bis 37 °C, die eine Chemisorption der Mikroorganismen aufgrund kovalenter und/oder ionischer Wechselwirkungen am Trägermaterial ermöglicht,
c) Abtrennung des mit den Mikroorganismen beladenen Trägermaterials von der Flüssigkeit,
d) gegebenenfalls Anreicherung des mit Mikroorganismen beladenen Trägermaterials in geeignetem Nährmedium,
e) Regenerierung des mit den Mikroorganismen beladenen Trägermaterials durch Lyse und gleichzeitige Isolierung einer DNA enthaltenden Lösung,
f) Analyse der DNA enthaltenden Lösung.

2. Verwendung nach Anspruch 1, **dadurch gekennzeichnet, dass** das Trägermaterial in Form eines Pulvers, eines Granulats, einer Folie, einer Membran, eines Sinterkörpers oder eines Schaums eingesetzt wird.

3. Verwendung nach einem der vorhergehenden Ansprüche, **dadurch gekennzeichnet, dass** das Trägermaterial hydrophil oder hydrophob funktionalisiert ist.

4. Verwendung nach einem der vorhergehenden Ansprüche, **dadurch gekennzeichnet, dass** die funktionellen Gruppen direkt oder über einen Spacer an das Trägermaterial gebunden sind.

5. Verwendung nach einem der vorhergehenden Ansprüche, **dadurch gekennzeichnet, dass** die Adsorption auf einer ionischen Wechselwirkung beruht und die funktionellen Gruppen ausgewählt sind aus der Gruppe bestehend aus Amin-, Carboxyl-, Carboxylat-, Sulfonsäure-, Sulfonat-, Phosphatgruppen und Proteine.

6. Verwendung nach einem der vorhergehenden Ansprüche, **dadurch gekennzeichnet, dass** die Adsorption auf einer kovalenten Wechselwirkung beruht und die funktionellen Gruppen ausgewählt sind aus der Gruppe bestehend aus Aktivester, Säureamide, Epoxide, Halogenide, Säurehalogenide und C-C-Mehrfachbindungen.

7. Verwendung nach einem der vorhergehenden Ansprüche, **dadurch gekennzeichnet, dass** das funktionalisierte Trägermaterial neutralen, kationischen oder anionischen Charakter besitzt.

8. Verwendung nach einem der vorhergehenden Ansprüche, **dadurch gekennzeichnet, dass** das Trägermaterial in dem flüssigen Medium dispergiert wird.

9. Verwendung nach einem der vorhergehenden Ansprüche, **dadurch gekennzeichnet, dass** das Trägermaterial in Form eines porösen Körpers vorliegt und das flüssige Medium dessen Poren durchströmt.

10. Verwendung nach einem der vorhergehenden Ansprüche, **dadurch gekennzeichnet, dass** die Abtrennung des Trägermaterials vom flüssigen Medium mittels Filtration, Sedimentation und/oder Absieben erfolgt.

11. Verwendung nach einem der vorhergehenden Ansprüche, **dadurch gekennzeichnet, dass** die Lyse mit einem Lysepuffer und/oder Enzymen erfolgt.

12. Verwendung nach vorhergehendem Anspruch, **dadurch gekennzeichnet, dass** die isolierten Mikroorganismen weiterbehandelt, insbesondere analysiert werden.

13. Verwendung nach einem der vorhergehenden Ansprüche, **dadurch gekennzeichnet, dass** die Mikroorganismen ausgewählt sind aus der Gruppe bestehend aus Bakterien, Hefen, Viren, Pilzen und Sporen.

## Claims

1. Use of a method for nonspecific separation and analysis of microorganisms from large-volume liquids, in which
a) in the liquid, a carrier material made of polymers, glass or ceramic, the surface of which was chemically functionalised for nonspecific adsorption of microorganisms contained in the liquid by binding of functional chemical groups, is distributed homgeneously, the pH value of the liquid being adjusted as a function of the type of microorganisms,
b) shaking of the liquid and of the carrier material at a temperature in the range of 10 to 37°C, which makes possible a chemisorption of the microorganisms on the basis of covalent and/or ionic interactions on the carrier material,
c) separation of the carrier material, which is loaded with the microorganisms, from the liquid,
d) if necessary, enrichment of the carrier material, which is loaded with microorganisms, in a suitable culture medium,
e) regeneration of the carrier material, which is loaded with the microorganisms, by means of lysis and simultaneous isolation of a DNA-comprising solution,
f) analysis of the DNA-comprising solution.

2. Use according to claim 1, **characterised in that** the carrier material is used in the form of a powder, a granulate, a film, a membrane, a sintered body or a foam.

3. Use according to one of the preceding claims, **characterised in that** the carrier material is functionalised hydrophilically or hydrophobically.

4. Use according to one of the preceding claims, **characterised in that** the functional groups are bonded directly or via a spacer to the carrier material.

5. Use according to one of the preceding claims, **characterised in that** the adsorption is based on an ionic interaction and the functional groups are selected from the group consisting of amine-, carboxyl-, carboxylate-, sulphonic acid-, sulphonate-, phosphate groups and proteins.

6. Use according to one of the preceding claims, **characterised in that** the adsorption is based on a covalent interaction and the functional groups are selected from the group consisting of active ester, acid amides, epoxides, halogenides, acid halogenides and C-C multiple bonds.

7. Use according to one of the preceding claims, **characterised in that** the functionalised carrier material has a neutral, cationic or anionic character.

8. Use according to one of the preceding claims, **characterised in that** the carrier material is dispersed in the liquid medium.

9. Use according to one of the preceding claims, **characterised in that** the carrier material is present in the form of a porous body and the liquid medium flows through the pores thereof.

10. Use according to one of the preceding claims, **characterised in that** the separation of the carrier material from the liquid medium is effected by means of filtration, sedimentation and/or sieving.

11. Use according to one of the preceding claims, **characterised in that** the lysis is effected with a lysis buffer and/or enzymes.

12. Use according to the preceding claim, **characterised in that** the isolated microorganisms are further treated, in particular analysed.

13. Use according to one of the preceding claims, **characterised in that** the microorganisms are selected from the group consisting of bacteria, yeasts, viruses, fungi and spores.

## Revendications

1. Utilisation d'un procédé de séparation non spécifique et d'analyse de microorganismes issus de liquides de grand volume, pour laquelle
a) un matériau support composé de polymères, de verre ou de céramique, dont la surface a été chimiquement fonctionnalisée, pour permettre une adsorption non spécifique de microorganismes contenus dans le liquide, par fixation de groupes chimiques fonctionnels, est réparti d'une manière homogène dans le liquide, le pH du liquide étant ajusté en fonction de l'espèce des microorganismes,
b) agitation du liquide et du matériau support à une température située dans la plage allant de 10 à 37 °C, qui permet une chimisorption des microorganismes sur la base d'interactions covalentes et/ou ioniques sur le matériau support,
c) séparation du matériau support chargé en les microorganismes du liquide,
d) le cas échéant, enrichissement, dans un milieu nutritif approprié, du matériau support chargé en microorganismes,
e) régénération du matériau support chargé en microorganismes, par lyse et simultanément isolement d'une solution contenant de l'ADN,
f) analyse de la solution contenant l'ADN.

2. Utilisation selon la revendication 1, **caractérisée en ce que** le matériau support est employé sous forme d'une poudre, d'un granulé, d'une feuille, d'une membrane, d'un corps fritté ou d'une mousse.

3. Utilisation selon l'une quelconque des revendications précédentes, **caractérisée en ce que** le matériau support a subi une fonctionnalisation hydrophile ou hydrophobe.

4. Utilisation selon l'une quelconque des revendications précédentes, **caractérisée en ce que** les groupes fonctionnels sont liés directement, ou par l'intermédiaire d'un espaceur, au matériau support.

5. Utilisation selon l'une quelconque des revendications précédentes, **caractérisée en ce que** l'adsorption repose sur une interaction ionique, et les groupes fonctionnels sont choisis parmi le groupe comprenant les groupes amine, carboxyle, carboxylate, acide sulfonique, sulfonate, phosphate et les protéines.

6. Utilisation selon l'une quelconque des revendications précédentes, **caractérisée en ce que** l'adsorption repose sur une interaction covalente, et les groupes fonctionnels sont choisis parmi le groupe comprenant les esters actifs, les amides d'acides, les époxydes, les halogénures, les halogénures d'acyle et les liaisons multiples C-C.

7. Utilisation selon l'une quelconque des revendications précédentes, **caractérisée en ce que** le matériau support fonctionnalisé possède un caractère neutre, cationique ou anionique.

8. Utilisation selon l'une quelconque des revendications précédentes, **caractérisée en ce que** le matériau support est dispersé dans le milieu liquide.

9. Utilisation selon l'une quelconque des revendications précédentes, **caractérisée en ce que** le matériau support se présente sous forme d'un corps poreux, et le milieu liquide s'écoule à travers ses pores.

10. Utilisation selon l'une quelconque des revendications précédentes, **caractérisée en ce que** la séparation du matériau support du milieu liquide s'effectue par filtration, sédimentation et/ou tamisage.

11. Utilisation selon l'une quelconque des revendications précédentes, **caractérisée en ce que** la lyse a lieu avec un tampon de lyse et/ou des enzymes.

12. Utilisation selon la revendication précédente, **caractérisée en ce que** les microorganismes isolés sont traités ultérieurement, en particulier analysés.

13. Utilisation selon l'une quelconque des revendications précédentes, **caractérisée en ce que** les microorganismes sont choisis parmi le groupe comprenant les bactéries, les levures, les virus, les champignons et les spores.
